# EUROPEAN PATENT SPECIFICATION

(11) **EP 2 043 569 B1**
(45) Date of publication and mention of the grant of the patent: **24.03.2010**
(21) Application number: 07809512.2
(22) Date of filing: 14.06.2007
(51) Int. Cl.: A61F 6/08

(54) **DIAPHRAGM HAVING A POLYMERIC SPRING MEMBER WITH NON-UNIFORM BENDING CHARACTERISTICS**
DIAPHRAGMA MIT POLYMERFEDERTEIL MIT UNGLEICHMÄSSIGER BIEGUNG
DIAPHRAGME AYANT UN ORGANE DE TYPE RESSORT POLYMÈRE À FLEXION NON UNIFORME

(30) Priority: 14.06.2006 US 804696 P
(43) Date of publication of application: 08.04.2009
(73) Proprietor: PATH, Seattle, WA 98107-5136 (US)
(72) Inventor: LABARRE, Paul, Suquamish, WA 98392 (US); PUAA, Kapaakea, C., Seattle, WA 98115 (US); AUSTIN, Glenn, Seattle, WA 98177 (US)
(74) Representative: Tombling, Adrian George
(86) International application number: PCT/US2007/013869
(87) International publication number: WO 2007/146333

(56) References cited:
- EP-A- 0 006 609
- DE-A1- 19 541 115
- GB-A- 961 880
- US-A- 2 811 153
- US-A- 5 228 456
- US-A- 5 771 900

## Description

The present invention is directed to female diaphragms, and more particularly to contraceptive and non-contraceptive diaphragms having a polymeric spring member with non-uniform bending characteristics and to methods for making and using the same.

### BACKGROUND

Contraceptive and non-contraceptive diaphragms, or simply diaphragms, have been used in their current form for over one hundred years. Contraceptive diaphragms are classified as an intra-vaginal barrier contraceptive and are commonly used in conjunction with spermicide.

Conventional contraceptive diaphragms are sized to fit wedged relatively tightly within the user's vagina. For example, the diaphragm known as the ORTHO ALL-FLEX diaphragm is supplied in 9 sizes (about 55 mm to 95 mm diameter in 5 mm increments) to fit the range of the female anatomy. In the United States, these medical devices have labeling restrictions such that they can only be purchased with a prescription from physician who has determined the appropriate size after following a fitting protocol.

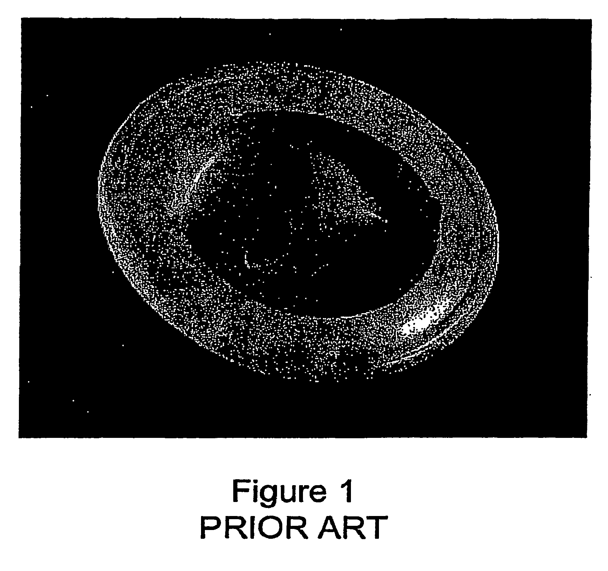

Figure 1 shows a conventional, planar contraceptive diaphragm. As illustrated, conventional diaphragms have a round shape formed by a generally planar loop having a diameter on the order of 55 to 95 mm, and a dome-shaped membrane which extends across the interior of the rim to form a barrier. The dome-shaped membrane is typically 0.5 mm thick and attached along a central, transverse axis of the rim. The rim and dome are usually made of latex rubber, but have been produced from other inert medical grade elastomers, such as silicone, urethane, vinyl, or thermoplastic elastomer (TPE). The rim has a generally cylindrical cross section which houses a spring that provides stiffness when folding or squeezing for insertion, and which opens the device after insertion. The spring is ordinarily in the form of either a single length of spring coil joined at its ends or a band of spring material.

In use, the diaphragm is inserted into the vagina so that the proximal end wedges in the posterior fornix behind the cervix and the distal end wedges behind the pubic notch. This wedged fit is intended to maintain coverage of the cervix during intercourse. However, there are a number of problems associated with the conventional diaphragm design. For instance, due to elongation of the vagina during sexual arousal and coitus, and axial movement of the cervix during various stages of the menstrual cycle, current diaphragms can move from their intended position leading to gaps around the edges of the diaphragm and compromising the barrier provided around the cervix. Accordingly, this type of contraceptive has suffered relatively high contraceptive failure rates of 13 to 17%. To enhance protection, diaphragm users are instructed to place a spermicidal material inside the dome and along the rim for contraceptive protection. Further, a number of various sizes are needed to fit the range of the female anatomy. This not only increases inventory concerns but also requires careful fitting by experienced clinicians in clinical settings. Users have also experienced difficulty in insertion and removal, discomfort including a false sense of "urgency" to urinate due to pressure on the urethra after extended wear, possible increased risk of urinary tract infections, discomfort for the sexual partner during coitus, unpleasant odor from the latex material over time, and deterioration of latex material over time if not carefully maintained.

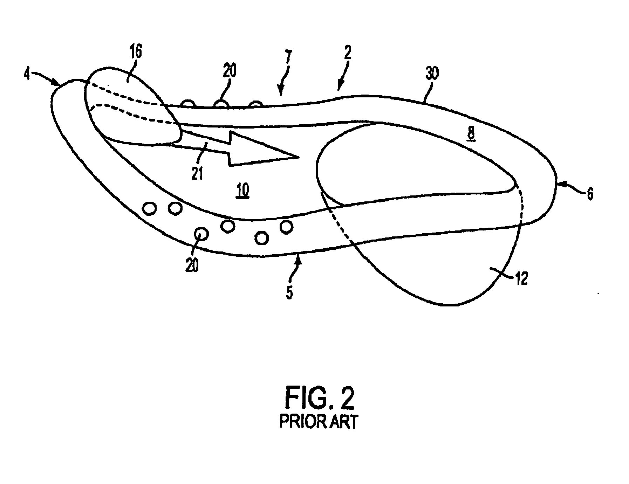

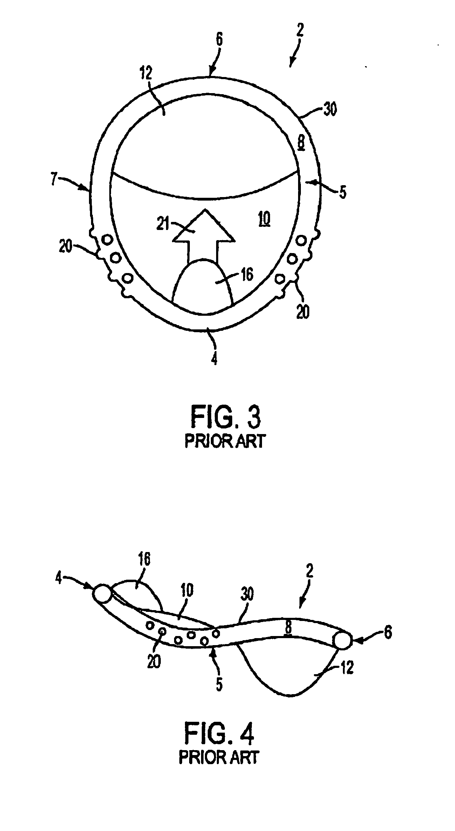

U.S. patent no. 5,771,900 issued June 30, 1998 to Austin et al. discloses a one-size contraceptive diaphragm (see Figure 2) that can fit women who would otherwise wear a fitted diaphragm within the range of traditional diaphragm sizes. An advantage of this device is that little or no physician interaction is required for fitting, which may permit it to be sold over the counter without the requirement for a prescription. As shown in Figure 4, diaphragm 2 shown in this patent has an anatomically contoured hoop-like rim 8 that surrounds a contoured membrane 10 forming two cup-like structures, a cervical dome 12 and finger dome 16. The larger dome 12 fits over the cervix during use and the smaller dome 16 can permit finger or thumb hooking of the rim for easy removal. U.S. patent no. 5,228,456 shows a female contraceptive device comprising the features of the preamble of claim 1.

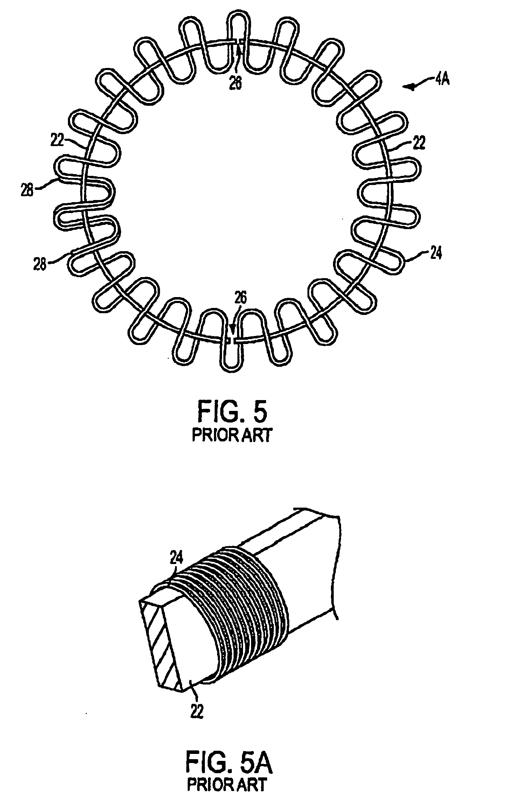

As shown in Figures 5 and 5A, rim 8 can be formed of two curvilinear flat bands 22, a spring 24, and a cover 30 (see Figure 2). The ends of each band abut the ends of the other band to form a loop. A single length open-coil compression spring 24 tightly surrounds the bands 22 to completely enclose the bands at their ends, and thereby form hinge joints 26. The spring 24 shown in Figure 5 is spread out for illustration of the overall construction. The spring 24, however, can be much more tightly wound about the bands to form a close coil or closed coil spring (Figure 5A). The bands can also be contoured such that the flats of the bands pitch (rotate) around the common center-line axis of the band, coil, and rim. The band and spring assembly is encased within cover 30. Cover 30 is generally a flexible tube-like member formed of microinjection molded medical-grade silicone rubber or other elastomeric material such as latex, TPE, urethane, vinyl or other suitable material. Cover 30 is typically premolded to match the shape of the spring and may have a cross section of various configurations including circular, elliptical, or rectangular with rounded corners.

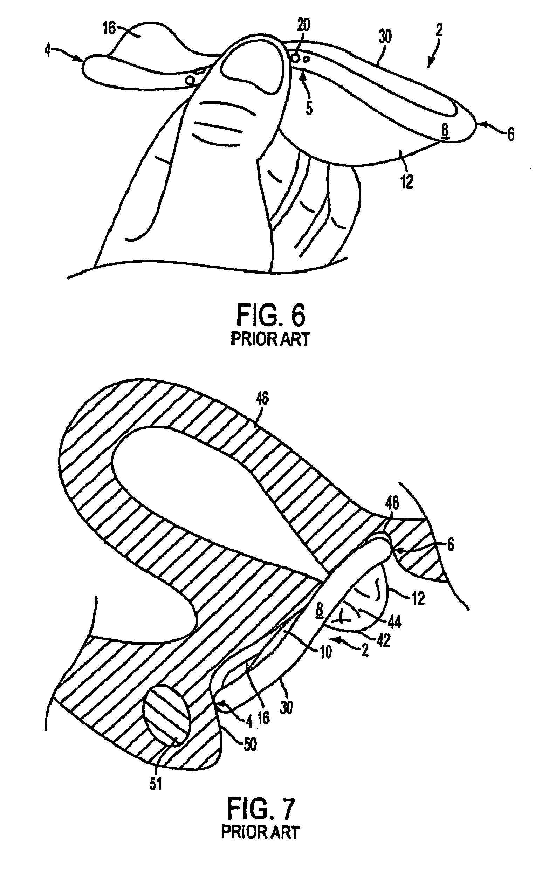

The cover, spring and band assembly of contraceptive diaphragm 2 can provide three dimensional contours and desired bending properties for rim 8. As shown in Figure 6, the bending dynamics caused by the contoured shape of rim 8 and the functioning of hinge joints 26 changes the shape of diaphragm 2 when squeezed by the user to improve the shape of the diaphragm for insertion. Its squeezed shape is more stable and the leading edge for insertion, that is posterior portion 6, is bent downwardly even more so that it more easily passes the anterior aspect of the cervix as the diaphragm is nestled into place. The interaction of coil spring 24 with hinge joints 26 allows diaphragm 2 to flex at the hinge joints to reduce the profile of the diaphragm, make its insertion less noticeable, and make its deployment within the vagina more effective. Once the diaphragm 2 is in place, coil spring 24 works with bands 22 to restore diaphragm 2 to its original shape. As shown in Figure 7, when in place, cervical dome 12 fits snugly around cervix 42 covering cervical os 44 of uterus 46. Posterior portion 6 fits snugly into posterior fornix 48.

Contraceptive diaphragm 2 provides many advantageous features. However, its spring and band assembly are difficult and relatively expensive to manufacture. The assembly requires skilled hand fabrication to properly assemble its multiple components. In addition, the components require very high tolerances for proper assembly, fit and operation.

### SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The principles of the invention may be advantageously used to provide a contraceptive or non-contraceptive diaphragm having a contoured shape and improved construction to facilitate insertion and removal, and to improve adherence of the diaphragm within the anatomy of a woman. In addition, the principles of the invention may be advantageously used to provide a contraceptive or non-contraceptive diaphragm having a rim with non-uniform bending properties to facilitate insertion, removal, and retention of the diaphragm, as well as to enhance performance and manufacturability of the diaphragm and to reduce its cost.

These and additional features and advantages of the invention will be described further below.

### BRIEF DESCRIPTION OF THE DRAWINGS

Figure 1 shows a conventional contraceptive diaphragm.

Figure 2 is a schematic perspective view of a contoured contraceptive diaphragm disclosed in U.S. patent no. 5,771,900 to Austin et al.

Figure 3 is a schematic top view of the contraceptive diaphragm of Figure 2.

Figure 4 is a schematic side view of the contraceptive diaphragm of Figure 2.

Figure 5 a schematic plan view of a pair of bands wrapped in a coil spring of the diaphragm of Figure 2.

Figure 5A is an enlarged portion of the bands illustrated in Figure 5.

Figure 6 is a schematic side view of a user grasping the contraceptive diaphragm of Figure 2.

FIG. 7 is a schematic partial section view showing the contraceptive diaphragm of Figure 2 during use.

Figure 8 is a schematic perspective view of a contoured contraceptive or non-contraceptive diaphragm according to aspects of the present invention.

Figure 9 is schematic perspective view of a first example configuration of a spring band of the diaphragm of Figure 8.

Figure 10 is another schematic perspective view of the diaphragm of Figure 8 shown with the spring band of Figure 9 being visible in the rim of the diaphragm.

Figure 11 shows a section of the spring band of Figure 9 that is generally positioned in region B of Figure 9.

Figure 12 shows a section of the spring band of Figure 12 that would generally be positioned in side region C shown in Figure 9.

Figure 13 shows a section of a second example configuration of a spring band for a section that would generally be positioned in region B shown in Figure 9.

Figure 14 shows a section of a second example configuration of a spring band for a section that would be positioned in anterior region C shown in Figure 9.

Figure 15 shows a section of the spring configuration of Figure 14 that would generally be positioned in region A shown in Figure 9.

Figure 16 is a close view of a central portion of the spring band section of Figure 14.

Figure 17 is schematic perspective view of a third example configuration of the spring band of Figure 8.

Figure 18 is a close view of a section of the spring band of Figure 17.

Figure 19 illustrates a method for manufacturing the diaphragm spring of Figure 17 according to aspects of the invention.

Figure 20 is a cross-sectional view taken along line Y-Y of Figure 19.

Figure 21 illustrates anticipated stress concentrations during insertion of the spring band of the diaphragm of Figure 8.

The figures referred to above are not drawn necessarily to scale and should be understood to present a simplified representation of the invention, illustrative of the basic principles involved. Some features of the diaphragm depicted in the drawings have been enlarged or distorted relative to others to facilitate explanation and understanding. The same reference numbers are used in the drawings for similar or identical components and features shown in various alternative embodiments. Diaphragms, as disclosed below, can have configurations and components determined, in part, by the intended application and environment in which they are used.

### DETAILED DESCRIPTION OF THE DRAWINGS

Unless otherwise stated, or otherwise clear from the context below, directional references used here are based on the orientation of components and assemblies shown in the appended drawings.

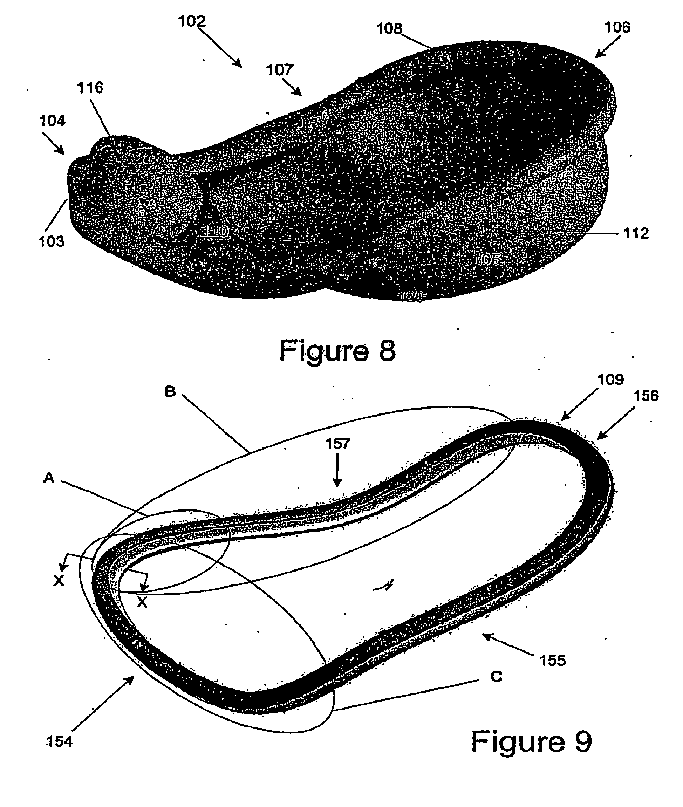

Figure 8 shows an embodiment of a contraceptive or non-contraceptive diaphragm 102 according to aspects of the invention. Diaphragm 102 is similar to the configurations of diaphragms 2 disclosed in U.S. patent no. 5,771,900 issued on June 30, 1998, which is incorporated herein by reference, except as discussed hereafter. However, it is understood that diaphragm 102 could be a contraceptive diaphragm or a non-contraceptive diaphragm. Further, it is understood that aspects of the invention could be used with both contraceptive and non-contraceptive diaphragms. For instance, diaphragms according to aspects of the invention could be provided for preventing sexually transmitted infections (STIs) and sexually transmitted diseases (STDs), collecting cervical fluid (menses collection), delivering a microbicide for STI prevention, delivering other medications to the cervix (e.g., cervical cancer treatment medications), delivering sperm to the cervix as part of artificial insemination procedures (human and animal), etc.

Diaphragm 102, having anterior portion 104, posterior portion 106, and lateral portions 105, 107, comprises a rim 108 which defines a loop that surrounds a membrane 110 and a cervical dome 112. Membrane 110 is preferably attached to rim 108 such that the plane of membrane 110 is substantially normal to rim 108 along its periphery. Although the membrane is relatively flat, it has some contour on account of its attachment to the rim which, as described below, has a curvilinear construction. The use of such a thin membrane composed of an elastomeric material enables the membrane to be extremely compliant in order to better cling to vaginal tissues when in use. Nevertheless, due to its thin construction and contoured shape, the diaphragm does not develop an overly aggressive suction.

Cervical dome 112 is a cup-shaped member which extends downwardly from membrane 110 in the posterior portion 106 of diaphragm 102. In an alternative configuration, the cervical dome may be offset slightly from the rim to aid the engagement of the diaphragm 102 with the posterior fornix. In either configuration, the dome opens upward to be received over the cervix and close the cervical os. Membrane 110 and dome 112 are preferably formed of silicone, but other suitable materials such as thermoplastic elastomer (TPE), latex, or urethane could be used. As shown, the anterior portion can include a flattened region 103, which improves contact with the vaginal wall during use to provide additional security created by this contact.

Rim 108 forms a loop which is preferably substantially elliptical in shape when viewed from above. As can be seen in Figure 9, the profile of rim 108 is not planar like that of a standard diaphragm. Rather, rim 108 has a curvilinear construction with changing radiuses in an axial direction. In one construction, rim 108 conforms to the shape of a conic projection. Rim 108 along anterior portion 104 of the diaphragm has a centerline contour which curves slightly upwardly thereby reducing interference with the partner and relieving pressure on the urethra that may occur with a standard round, planar ring rim. Rim 108 along posterior portion 106 can have a centerline contour which curves slightly downwardly thereby providing for better fit in the fornix surrounding the proximal portion of the cervix. The contours of the rim 108 allow the lateral anterior quadrants of the diaphragm 102 to securely engage supportive tissue in the vagina via shallow embedment in the vaginal wall adjacent to supportive muscle or connective tissue. The gentle arcs of the rim along anterior and posterior portions 104, 106 also provide better engagement, or clinging, of the thin membrane 110 to the vaginal tissue.

A single diaphragm 102 can replace five or more sizes of standard diaphragms since its shape and construction allows it to advantageously conform and cling to the anatomy of the vagina. The diameter, or size, of one preferred embodiment of diaphragm 102 is 70 mm which would typically fit women who use standard diaphragms having sizes of 65 mm to 85 mm.

Diaphragm 102 has a reduced bulk when compared to standard diaphragms due to the fact that cervical dome 112 extends from only the posterior portion 106 of the diaphragm whereas the domes of a standard diaphragm extends across the entire rim. The reduced bulk and contoured shape of diaphragm 102 advantageously aid overall handling, increase reliability, reduce stress on the cervix when in position, make the diaphragm easier to store and more inviting to wear, increase partner acceptability, and ease insertion and removal of the diaphragm into and from the introitus. Such a construction also lowers production costs due to the reduced volume of material used.

Diaphragm 102 can also include a cup shaped projection or finger dome 116 opening downwardly from membrane 110 in the anterior portion 104 of the diaphragm. Finger dome 116 is provided to ease removal of the diaphragm. Finger dome 116 can be located adjacent the anterior vaginal wall slightly behind pubic bone such that it can be easily grasped or hooked with a finger for removal. Also, since the finger dome is composed of pliable material, the dome tends to collapse and thereby conform to the woman's internal anatomical structure. As discussed above, the upwardly curving anterior portion 104 lifts rim 108 helping to avoid an obstruction for the wearer's partner and relieving pressure on the urethra. To remove diaphragm 102, a finger is inserted into finger dome 116 and the diaphragm is then pulled from the vagina. This embodiment may provide less interference with vaginal anatomy in smaller size vaginas, i.e., those of women normally wearing 60 mm to 70 mm diaphragms. As shown, the finger dome may be placed offset from the anterior centerline proximate flattened region 103.

As further shown in Figure 8, small dome shaped projections 120 may be provided along a portion of lateral portions 105, 107 of rim 108. Projections 120 preferably have a height of 0.5-2.5 mm and a diameter of 1.0-3.0 mm, and are most preferably 1.0 mm high with a diameter of 2 mm. Projections 120 provide a tactile indication of the optimal location for grasping the diaphragm 102 as well as improved grip when inserting the diaphragm into the vagina, especially when it is made slippery by the application of spermicide, gel or other lubricants.

When inserting diaphragm 102, which is generally illustrated in Figure 6 using similar diaphragm 2, it is grasped about lateral portions 105, 107 and squeezed. The bending dynamics caused by the contoured shape of rim 108 and the selected bending dynamics discussed below for spring band 109 changes the shape of diaphragm 102, thereby optimizing the shape of the diaphragm for insertion. Its squeezed shape is more stable and the leading edge for insertion, that is posterior portion 106, is bent downwardly even more so that it more easily passes the anterior aspect of the cervix as the diaphragm is nestled into place. The bending dynamics of spring band 109 allows diaphragm 102 to flex at desired hinge locations thereby reducing the profile of the diaphragm and making its insertion less noticeable and making its deployment within the vagina more effective. Once the diaphragm 102 is in place, spring band 109 restores diaphragm 102 to its original shape.

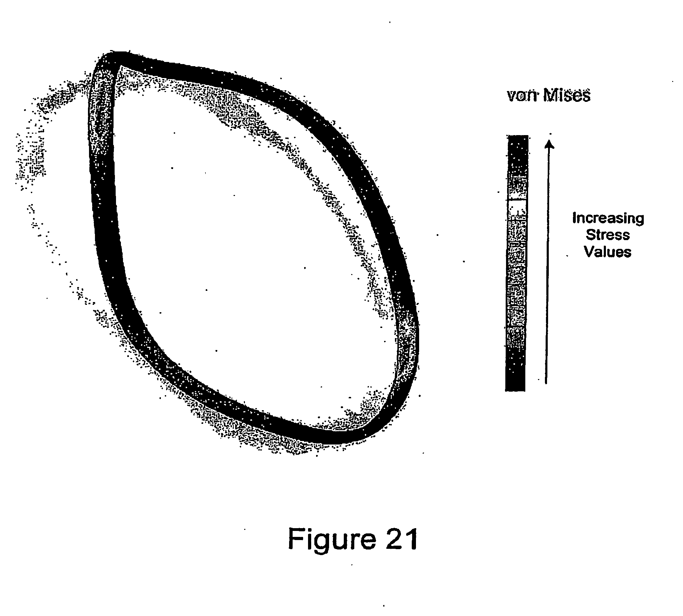

Rim 108 preferably includes an engineering thermoplastic or thermoset polymeric material spring band 109 that acts as a spring member to improve insertability of the diaphragm and to improve retention of the rim in its contoured configuration. In general, polymeric band 109 provides the functional advantages as the assembly of bands 22 and spring 24 discussed in U.S. patent number 5,771,900. However, polymeric band 109 provides a spring member that has various additional advantages as described herein, such as increased design flexibility, improved manufacturability, and reduced cost. As shown in Figures 9 and 11-18, spring band 109 can have a generally elliptical or oval cross-section. The orientation, thickness and other characteristics of its elliptical cross-section can change along its length to provide varying bending and spring characteristics. The configurations shown in Figures 9 and 11-18 have been prototyped and tested for strength and stiffness as well as other bending characteristics. Figure 21 shows the results of finite element analysis calculations for spring band 109 when the diaphragm is in a folded insertion configuration similar to the configuration shown in Figure 6. The areas shown in red are high stress areas and the areas in yellow/green are moderate stress areas.

Spring band 109, formed as a polymeric member, has varying geometries that provides varying spring characteristics. The use of an elastomer to form spring band 109 provides advantages such as permitting the spring band to have a desired contour and to have varying geometries. Figures 9 and 11-18 illustrate various polymeric spring bands 109 having different configurations. However, each of these configurations has anterior and posterior sections 106 and 103 that are canted according to the desired contour of the diaphragm 102.

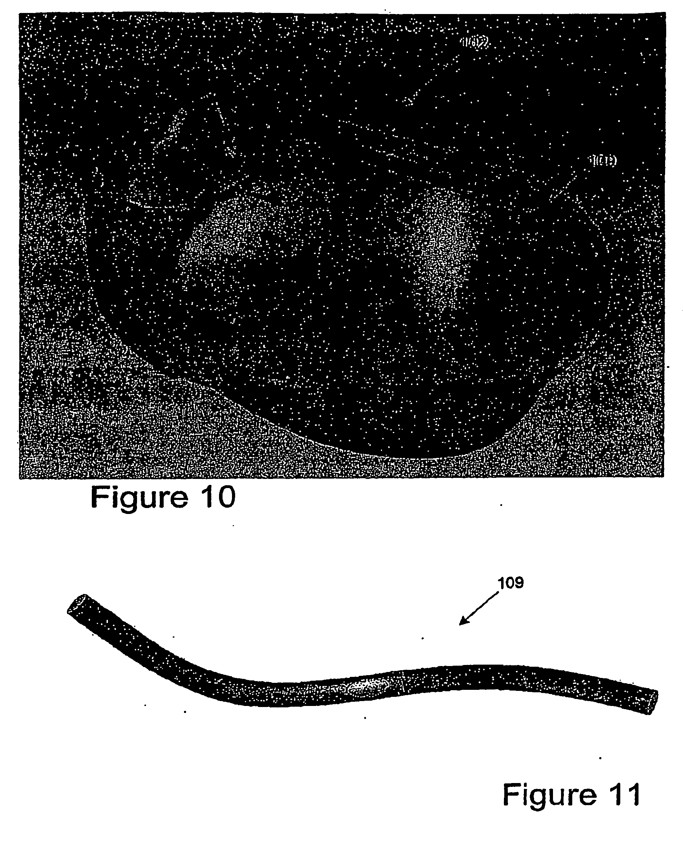

The configuration of Figures 9 and 11 has an elliptical cross section that is uniform in both size and orientation along its length. Such a configuration is relatively easy to manufacture due to its uniformity. However, it lacks the ability to favor bending in certain areas and in particular directions and to provide increased spring force in other areas.

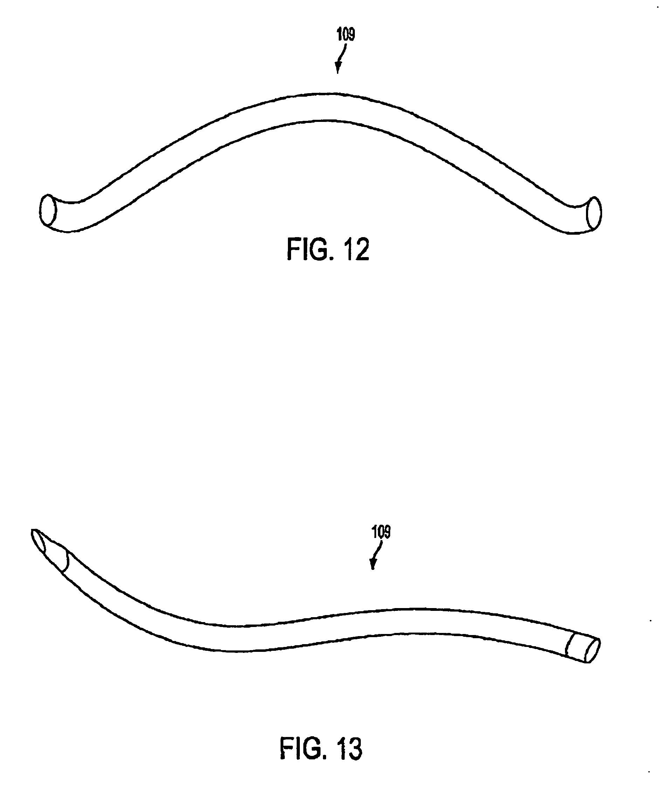

The configuration of Figures 12 and 13 illustrates a second example configuration in which the cross sectional width at the anterior section 106 and the posterior section 103 (Figure 9) are tapered to be half the cross sectional width of the lateral portions 105 and 107. As such, the anterior and posterior sections bend more easily than the lateral portions to encourage the diaphragm to fold together into an insertion configuration similar to the configuration shown in Figure 6. The improved bending at the anterior and posterior sections reduces stress values in the high stress areas shown in Figure 21. As further shown, the orientation of the ellipse can change (e.g., twists) to improve bending and resiliency in certain directions at varying points along the band. For instance, the ellipse is vertically oriented at the anterior and posterior sections so that it can more easily bend at the thinner portion of the ellipse.

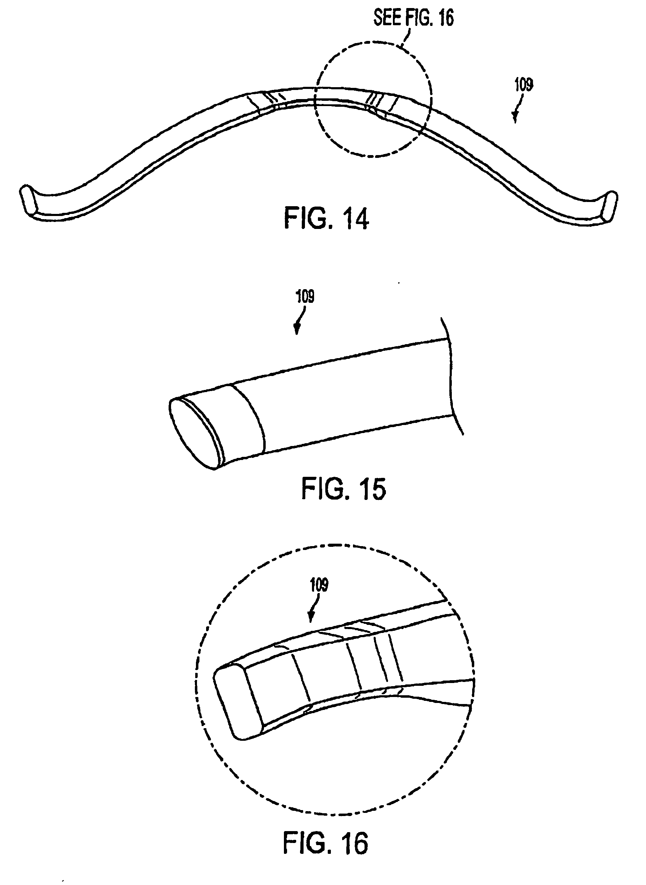

The configuration of Figures 14-16 illustrates a third example configuration in which the anterior portion of the spring band necks down to have a thinner elliptical cross section at a desired bending location. Figure 16 shows a portion of the band where it necks down to form an elongated ellipse. The necked down portions in the anterior and posterior sections permit the spring band to easily bend at those portions during insertion of the diaphragm. In addition, the oval cross-section at the anterior and posterior sections can provide for improved mold manufacturability since larger radius cutting tools can be used to cut the spring profile into the mold.

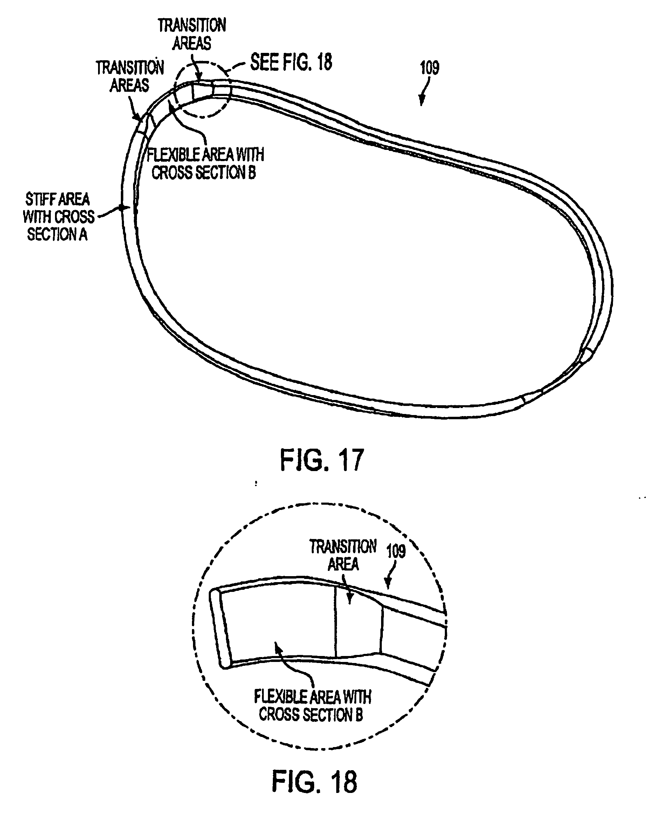

Figures 17 and 18 show a fourth example configuration of spring band 109, which is generally a preferred configuration for use with diaphragm 102. The spring band in this configuration is generally the same as the spring band shown in Figures 14-16, except that area B has elongated cross sections such that the length of their elliptical cross sections are longer than the length of the cross sections to either side (e.g., cross section A). The elongated sections provide larger hinges to improve fatigue life of the spring band.

The configuration of Figures 17 and 18 provide a spring band having a constant cross section A along its sides whose area moment of inertia creates a stiff region along that portion of the spring band. It also has a constant cross section B at the ends whose area moment of inertia creates a flexible region along the spring to promote the majority of bending in this region. Both cross section A and B are oval with a major axis and a minor axis. Thus the moment of inertia at any given point along the spring favors greater bending in one plane of the cross section than the other. Additionally, there is a transition region between cross sections A and B through which the cross section maintains an oval shape, but the major and minor diameters smoothly transition from cross section A to B or vice versa. The orientation of the oval at any given point is chosen to promote specific bending properties of the diaphragm to aid with easy insertion and removal and to provide a comfortable fit.

In general, spring band 109 has a varying cross section, which influences the bending dynamics of rim 108. As discussed above, spring band 109 can be a non-circular, non-coiled, continuous, non-planar hoop with oval cross-sections of varying major and minor diameter. Spring bands according to aspects of the invention described along with the above configurations provide various advantageous bending properties. For example, improved bending at the anterior portion can permit the diaphragm to be relatively small at the end inserted into the vagina to, thereby, promote easy insertion. Therefore the radius of the forward edge of the diaphragm when bent is relatively small, but is blunt enough to avoid inducing discomfort or injury. In addition, the insertion and removal ends of the diaphragm are permitted to drop when the diaphragm is squeezed for insertion to create a streamlined device for insertion. When the diaphragm unbends in the vagina, the spring band 109 induces forces on the rim and within the vagina to promote a secure fit of the device such that the diaphragm dome covers the cervix, the insertion end is tucked into the posterior fornix and the removal end is tucked behind the pubic bone.

Spring band 109 can be formed from a variety of polymers having appropriate characteristics, such as biocompatibility, strength, stiffness, and elongation. For example, the spring can be formed from injection molded or cast medical-grade silicone rubber, nylon, acetal, polyphenylsulfone, PET, PBT or other suitable material. Suitably robust materials can be selected based on biocompatibility, polymer class, manufacturing temperatures, flexural modulus, fatigue resistance and elongation at yield. The preferable material for spring band 109 is a Nylon 6,6 known as SOLUTIA VYDYNE 21SP. This resin was selected as the preferred material for use with diaphragm 102 based on the criteria above and the results of the following tests using this material with the above described configurations of spring band 109.
- Short axis compression: the diaphragm sides were compressed on a force/displacement test machine in a manner similar to that used during insertion into the vagina.
- Long axis compression: the diaphragm anterior and posterior sections were compressed on a force/displacement test machine to simulate forces exerted on the device by the posterior fornix and pubic arch while the device is in the vagina.
- Resistance to boat bending: the stability of the device or resistance to unacceptable bending that might lead to incomplete cervical coverage when the device is in the vagina was assessed.
- Kneel-down performance: the dynamic behavior of the anterior and posterior, which ideally "kneels" or drops down during short axis compression to promote easier insertion was assessed.
- Relaxation behavior: the material properties of the spring and resistance to creep over time when under a load was assessed.
- Water absorption: Since the diaphragm device would be subjected to periodic cleaning, tests were conducted to determine to what extent water absorption affected the spring performance.

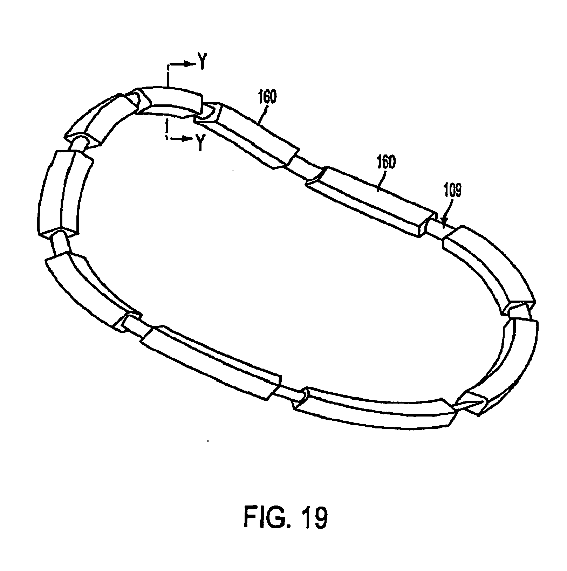

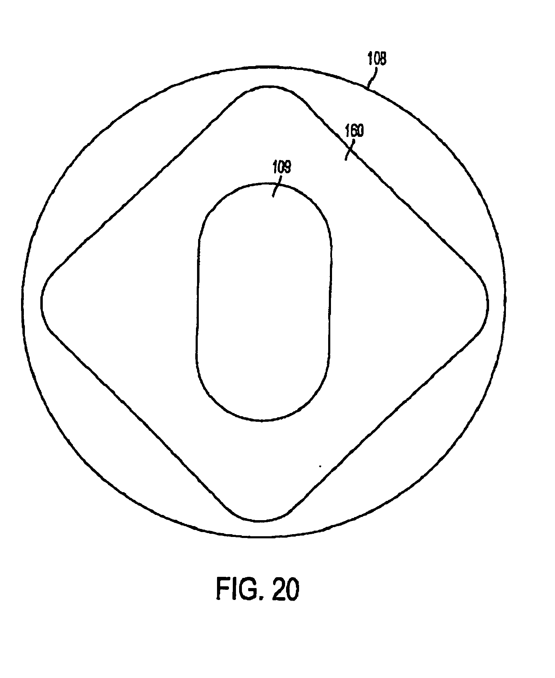

Figures 19 and 20 illustrate a method for manufacturing spring band 109 in accordance with the invention. As shown in Figure 19, a series of segmented spacers 160 are molded onto the spring band prior to its final over-molding in the diaphragm tool. The configuration of these spacers, including the number of spacers and their shape, can be varied as desired. The shape and number of these spacers are preferably sufficient to promote centering of the spring band cross section within the rim cross section and to promote adequate flow of liquid silicone resin between segments. Figure 20 shows one preferred configuration of the spring band, spacer and rim. As shown, the configuration includes a preferred gap of 0,25 mm (0.01 inches) or more between the rim and the inside of the mold, which provides a rim thickness of 0,25mm (0.01 inches) proximate to the spacers.

In light of the foregoing disclosure of the invention and description of certain preferred embodiments, those who are skilled in this area of technology will readily understand that various modifications and adaptations can be made without departing from the scope of the invention. All such modifications and adaptations are intended to be covered by the following claims.

## Claims

1. A female contraceptive device comprising:
a rim forming a first loop;
a barrier portion extending across said loop, said barrier portion including a cervical dome which opens in a first axial direction; and
a polymeric spring member within said rim forming a second loop within said first loop;
**characterized by**
said polymeric spring member being a non-circular, non-coiled, continuous non-planar hoop having oval cross-sections of varying major and minor diameter and non-uniform bending characteristics.

2. A female contraceptive device in accordance with claim 1, wherein said oval cross-sections of varying major and minor diameter promote the non-uniform bending characteristics.

3. A female contraceptive device in accordance with claim 1, wherein the polymeric spring member has reduced cross section portions to favor bending at the reduced cross section portions.

4. A female contraceptive device in accordance with claim 1, wherein the polymeric spring member is formed from one or more thermoplastic or thermoset polymeric materials.

5. A female contraceptive device in accordance with claim 1, wherein the barrier portion is adapted to be oriented towards a user's cervix during use, and said non-uniform bending characteristics of the polymeric spring member favor bending for collapsing said second loop while resisting bending in a second axial direction opposite said first axial direction.

6. A female contraceptive device in accordance with claim 3, wherein at least one of said reduced cross section portions favors bending in a first direction while resisting bending in a second direction.

7. A female contraceptive device in accordance with claim 4, wherein said one or more thermoplastic or thermoset polymeric materials are homogeneous throughout said polymeric spring member.

8. A female contraceptive device in accordance with claim 1, wherein a contour of said rim in a relaxed state conforms to the contour of the user's cervix.

9. A female contraceptive device in accordance with claim 8, wherein said rim curves in the first axial direction at an anterior end and curves in an opposite second axial direction at an opposite posterior end.

10. A female contraceptive device in accordance with claim 1, wherein said cervical dome opens in a first axial direction adapted to be oriented towards a user's cervix during use, and said polymeric spring member has a changing area moment of inertia along its length providing non-uniform resistance to bending.

11. A female contraceptive device in accordance with claim 10, wherein said changing area moment of inertia is configured to resist bending in a second axial direction opposite said first axial direction.

12. A female contraceptive device in accordance with claim 11, wherein said changing area moment of inertia is configured to favor bending for collapsing said second loop.

13. A female contraceptive device in accordance with claim 10, wherein said polymeric spring member has a non-uniform cross-section providing said changing area moment of inertia.

14. A female contraceptive device in accordance with claim 1, wherein said non-uniform bending characteristics of the polymeric spring favor bending for collapsing said second loop while resisting bending in a second axial direction opposite said first axial direction, said polymeric spring member comprising:
an anterior region curving in said first axial direction;
a posterior region opposite the anterior region curving in a second axial direction opposite said first axial direction;
one or more thermoplastic or thermoset homogeneous polymeric materials forming said polymeric spring member; and
a plurality of reduced cross section portions formed in the polymeric spring member each favoring bending in a first direction while resisting bending in a second direction.

## Patentansprüche

1. Empfängnisverhütungsmittel für Frauen, umfassend
einen Rand, der eine erste Schleife bildet,
einen Barrierenabschnitt, der sich quer durch die Schleife erstreckt, wobei der Barrierenabschnitt einen zervikalen Dom aufweist, der in eine erste axiale Richtung öffnet, und
ein Polymer-Federelement innerhalb des Rands, das eine zweite Schleife innerhalb der ersten Schleife bildet,
**dadurch gekennzeichnet, dass**
das Polymer-Federelement ein nicht kreisförmiger, nicht spiraliger und kontinuierlicher nicht ebener Reifen ist, der ovale Querschnitte mit verschiedenem größerem und kleinerem Durchmesser und nicht einheitlichen Biegecharakteristiken aufweist.

2. Empfängnisverhütungsmittel für Frauen nach Anspruch 1, wobei die ovalen Querschnitte mit verschiedenem größerem und kleinerem Durchmesser die nicht einheitlichen Biegecharakteristiken fördern.

3. Empfängnisverhütungsmittel für Frauen nach Anspruch 1, wobei das Polymer-Federelement reduzierte Querschnittsabschnitte aufweist, um das Biegen an den reduzierten Querschnittsabschnitten zu begünstigen.

4. Empfängnisverhütungsmittel für Frauen nach Anspruch 1, wobei das Polymer-Federelement aus einem oder mehreren thermoplastischen oder duroplastischen Polymermaterialien ausgebildet ist.

5. Empfängnisverhütungsmittel für Frauen nach Anspruch 1, wobei der Barrierenabschnitt derart ausgebildet ist, dass er während der Verwendung zum Gebärmutterhals einer Benutzerin orientiert ist, und die nicht einheitlichen Biegecharakteristiken des Polymer-Federelements das Biegen zum Einfalten der zweiten Schleife begünstigen, während einem Biegen in eine zweite axiale Richtung, die der ersten axialen Richtung entgegengesetzt ist, entgegengewirkt wird.

6. Empfängnisverhütungsmittel für Frauen nach Anspruch 3, wobei zumindest einer der reduzierten Querschnittsabschnitte ein Biegen in eine erste Richtung begünstigt, während einem Biegen in eine zweite Richtung entgegengewirkt wird.

7. Empfängnisverhütungsmittel für Frauen nach Anspruch 4, wobei das eine oder die mehreren thermoplastischen oder duroplastischen Polymermaterialien homogen in dem Polymer-Federelement vorliegen.

8. Empfängnisverhütungsmittel für Frauen nach Anspruch 1, wobei eine Kontur des Rands in einen entspannten Zustand mit der Kontur des Gebärmutterhalses der Nutzerin übereinstimmt.

9. Empfängnisverhütungsmittel für Frauen nach Anspruch 8, wobei der Rand an einem vorderen Ende in die erste axiale Richtung gekrümmt ist und an einem entgegengesetzten hinteren Ende in eine entgegengesetzte zweite axiale Richtung gekrümmt ist.

10. Empfängnisverhütungsmittel für Frauen nach Anspruch 1, wobei der zervikale Dom in eine erste axiale Richtung öffnet und dazu angepasst ist, während der Verwendung zu dem Gebärmutterhals einer Nutzerin orientiert zu sein, und wobei das Polymer-Federelement ein wechselndes Flächenträgheitsmoment entlang seiner Länge aufweist, welches den nicht einheitlichen Biegewiderstand bereitstellt.

11. Empfängnisverhütungsmittel für Frauen nach Anspruch 10, wobei das wechselnde Flächenträgheitsmoment derart ausgestaltet ist, dass es einem Biegen in eine zweite axiale Richtung entgegenwirkt, die der ersten axialen Richtung entgegengesetzt ist.

12. Empfängnisverhütungsmittel für Frauen nach Anspruch 11, wobei das wechselnde Flächenträgheitsmoment derart ausgestaltet ist, dass ein Biegen zum Falten der zweiten Schleife begünstigt ist.

13. Empfängnisverhütungsmittel für Frauen nach Anspruch 10, wobei das Polymer-Federelement einen nicht einheitlichen Querschnitt aufweist, der das wechselnde Flächenträgheitsmoment bereitstellt.

14. Empfängnisverhütungsmittel für Frauen nach Anspruch 1, wobei die nicht einheitlichen Biegecharakteristiken des Polymer-Federelements ein Biegen zum Einfalten der zweiten Schleife begünstigen, während sie einem Biegen in eine zweite axiale Richtung entgegenwirken, die der ersten axialen Richtung entgegengesetzt ist, und wobei das Polymer-Federelement umfasst:
eine vordere Region, die in die erste axiale Richtung gekrümmt ist,
eine hintere Region, die der vorderen Region entgegengesetzt ist und in eine zweite axiale Richtung gekrümmt ist, die der ersten axialen Richtung entgegengesetzt ist,
ein oder mehrere thermoplastische oder duroplastische homogene Polymermaterialien, die das Polymer-Federelement bilden, und
eine Vielzahl von reduzierten Querschnittsabschnitten, die in dem Polymer-Federelement ausgebildet sind und jeweils ein Biegen in eine erste Richtung begünstigen, während sie einem Biegen in eine zweite Richtung entgegenwirken.

## Revendications

1. Dispositif contraceptif féminin comprenant :
un bord formant une première boucle ;
une partie de barrière s'étendant sur ladite boucle, ladite partie de barrière comprenant un dôme cervical qui s'ouvre dans une première direction axiale ; et
un élément de ressort polymère à l'intérieur dudit bord formant une seconde boucle à l'intérieur de ladite première boucle ;
**caractérisé par** :
ledit élément de ressort polymère étant un cercle non circulaire, non hélicoïdal, continu non plan ayant des sections transversales ovales de diamètres majeur et mineur variables et des caractéristiques de flexion non uniformes.

2. Dispositif contraceptif féminin selon la revendication 1, dans lequel lesdites sections transversales ovales des diamètres majeur et mineur variables favorisent les caractéristiques de flexion non uniformes.

3. Dispositif contraceptif féminin selon la revendication 1, dans lequel l'élément de ressort polymère a des parties de section transversale réduites afin de favoriser la flexion au niveau des parties de section transversale réduites.

4. Dispositif contraceptif féminin selon la revendication 1, dans lequel l'élément de ressort polymère est formé à partir d'un ou de plusieurs matériaux thermoplastiques ou polymères thermodurcissables.

5. Dispositif contraceptif féminin selon la revendication 1, dans lequel la partie de barrière est adaptée pour être orientée vers le col de l'utérus d'une utilisatrice pendant l'usage, et lesdites caractéristiques de flexion non uniformes de l'élément de ressort polymère favorisent la flexion pour replier ladite seconde boucle tout en résistant à la flexion dans une seconde direction axiale opposée à ladite première direction axiale.

6. Dispositif contraceptif féminin selon la revendication 3, dans lequel au moins l'une desdites parties de section transversale réduites favorise la flexion dans une première direction tout en résistant à la flexion dans une seconde direction.

7. Dispositif contraceptif féminin selon la revendication 4, dans lequel lesdits un ou plusieurs matériaux thermoplastiques ou polymères thermodurcissables sont homogènes tout le long dudit élément de ressort polymère.

8. Dispositif contraceptif féminin selon la revendication 1, dans lequel un contour dudit bord dans un état au repos se conforme au contour du col de l'utérus de l'utilisatrice.

9. Dispositif contraceptif féminin selon la revendication 8, dans lequel ledit bord s'incurve dans la première direction axiale au niveau d'une extrémité antérieure et s'incurve dans une seconde direction axiale opposée au niveau d'une extrémité postérieure opposée.

10. Dispositif contraceptif féminin selon la revendication 1, dans lequel ledit dôme cervical s'ouvre dans une première direction axiale adaptée pour être orientée vers le col de l'utérus d'une utilisatrice et ledit élément de ressort polymère a une zone de moment d'inertie variable le long de sa longueur fournissant la résistance non uniforme à la flexion.

11. Dispositif contraceptif féminin selon la revendication 10, dans lequel la zone de moment d'inertie variable est configurée pour résister à la flexion dans une seconde direction axiale opposée à ladite première direction axiale.

12. Dispositif contraceptif féminin selon la revendication 11, dans lequel ladite zone de moment d'inertie variable est configurée pour favoriser la flexion afin de replier ladite seconde boucle.

13. Dispositif contraceptif féminin selon la revendication 10, dans lequel ledit élément de ressort polymère a une section transversale non uniforme fournissant ladite zone de moment d'inertie variable.

14. Dispositif contraceptif féminin selon la revendication 1, dans lequel lesdites caractéristiques de flexion non uniformes du ressort polymère favorisent la flexion pour replier ladite seconde boucle tout en résistant à la flexion dans une seconde direction axiale opposée à ladite première direction axiale, ledit élément de ressort polymère comprenant :
une région antérieure s'incurvant dans ladite première direction axiale ;
une région postérieure opposée à la région antérieure s'incurvant dans une seconde direction axiale opposée à ladite première direction axiale ;
un ou plusieurs matériaux thermoplastiques ou polymères homogènes thermodurcissables formant ledit élément de ressort polymère ; et
une pluralité de parties de section transversale réduites formées dans l'élément de ressort polymère favorisant chacune la flexion dans une première direction tout en résistant à la flexion dans une seconde direction.
